# EUROPEAN PATENT SPECIFICATION

(11) **EP 4 103 942 B1**
(45) Date of publication and mention of the grant of the patent: **09.09.2026**
(21) Application number: 21753721.6
(22) Date of filing: 11.02.2021
(51) Int. Cl.: C07C 67/08, G01N 30/86, G01N 31/00, G16C 20/20, C07C 69/78, A61K 8/37, A61Q 19/00

(54) **NATURAL C12-15 ALKYL BENZOATES**
NATÜRLICHE C12-15-ALKYLBENZOATE
BENZOATES D'ALKYLE EN C12-15 NATURELS

(30) Priority: 11.02.2020 US 202062975026 P
(43) Date of publication of application: 21.12.2022
(73) Proprietor: HallStar Beauty and Personal Care Innovations Company, Chicago, IL 60606 (US)
(72) Inventor: DAVIS, Brian, Chicago, Illinois 60606 (US); NEUDAHL, Gary, Chicago, Illinois 60606 (US); ZHANG, Jean, Chicago, Illinois 60606 (US)
(74) Representative: Kilger, Ute
(86) International application number: PCT/US2021/017625
(87) International publication number: WO 2021/163301

(56) References cited:
- EP-B1- 2 010 148
- KR-A- 20140 060 756
- US-A1- 2010 028 275
- US-A1- 2010 028 275
- AL-MASSAEDH “AYAT ALLAH”; PYELL UTE: "Preparation of Cationic Mixed-Mode Acrylamide-Based Monolithic Stationary Phases for Capillary Electrochromatography", CHROMATOGRAPHIA, vol. 81, no. 9, 30 June 2018 (2018-06-30), pages 1325 - 1336, XP036572457, ISSN: 0009-5893, DOI: 10.1007/s10337-018-3564-7
- ATSUO NAKAE , GIICHI MUTO: "Chromatographic behaviour of alkylbenzenes and alkyl benzoates on porous micro-spherical poly(styrene-divinylbenzene) gel", JOURNAL OF CHROMATOGRAPHY A, vol. 120, no. 1, 12 May 1976 (1976-05-12), pages 47 - 54, XP055847067, ISSN: 0021-9673, DOI: 10.1016/S0021-9673(01 )98996-4

## Description

US 2010/028275 A1 describes alkylbenzoate mixtures, the sum of the C12- and C14-alkylbenzoates being greater than or equal to 85%, in relation to the total sum of the alkylbenzoates. Described is also the use of said alkylbenzoate mixtures in cosmetic and/or pharmaceutical preparations, in particular as oil components.

Disclosed are novel C12-15 Alkyl Benzoates, as specified in claim 1, that are made in part from natural sources, i.e. from specific palm-based alkyl compounds having chain lengths in the range of 12 to 15 carbon atoms, e.g., palm-based C12 compounds, that are useful for a full array of cosmetic uses, including in all known uses for known C12-15 Alkyl Benzoates, including FINSOLV^{®} TN by Innospec. Furthermore, the novel C12-15 Alkyl Benzoates provide a large number of advantages over the known product in the art, including costs savings as well as providing a more natural product, which would be more desirable especially in the fields of uses of cosmetics, and beauty and/or body care products as well as in fragrances, where naturally sourced materials are typically highly valued by customers.

In particular, an aspect of the invention is an alkyl benzoate composition,
having peak area ratios for five main peaks by HPLC @ 230nm within a tolerance of ± 10% as follows

**[Table 1]**

| peak# | peak area ratio |
|---|---|
| 1 | 47.4 |
| 2 | 9.5 |
| 3 | 23.4 |
| 4 | 7.2 |
| 5 | 3.8 |

wherein the alkyl benzoate composition is produced by a process comprising:
charging a batch with 43% palm-based 12/70 alcohol and 13.5% synthetic C12-C15 alcohol and 45% benzoic acid,
bringing the batch to a predetermined temperature,
introducing a catalyst at the predetermined temperature,
and cooling the batch once free alcohol content is 0.5% or less.

In an embodiment of the invention, to the composition of the invention C15 alkyl benzoate compounds are added.

In an embodiment of the invention, the tolerance is ±5%.

In an embodiment of the invention, the tolerance is ±1%.

A further aspect of the invention is a cosmetics, or beauty and/or body care product or fragrance, comprising the composition according to the invention.

The novel C12-15 Alkyl Benzoates can be used, e.g., as emollients, solubilizer, wetting agent and/or anti-tack agent, in a large variety of cosmetic and/or beauty and/or body care products including sunscreens, suntans, deodorants, antiperspirants, emollient creams, hand creams and/or lotions, as well as in body creams and/or lotions, body and massage oils, alcoholic lotions, make-ups and/or nail products, e.g., foundation lotions, lipsticks, lip glosses, lip balms, make-up removers, face masks, bath and after bath products, shaving preparations, pre-electric shave lotions, syndets, hair care products, powders, fragrances, foot care products and after shave lotions and/or balms.

The novel C12-15 Alkyl Benzoates prepared from palm-based alkyl groups possess non-toxic, non-irritating, non-sensitizing and non-comedogenic properties as do other known C12-15 Alkyl Benzoates.

These C12-15 Alkyl Benzoate compounds can be generally described by the following general formula 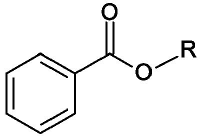 wherein R is C12 to C15 alkyl, preferably linear, but branching is possible, preferably non-substituted, but substituents are possible, e.g., one or more OH groups, e.g., at the terminal carbon atom of the alkyl group.

The novel C12-15 Alkyl Benzoate compounds can be in a mixture of such C12-15 Alkyl Benzoate compounds with certain amount of the compounds being C12 Alkyl Benzoate compounds, C13 Alkyl Benzoate compounds, C14 Alkyl Benzoate compounds and/or C15 Alkyl Benzoate compounds. The amount of each type of compound in a mixture affects the characteristics of the mixture. Such novel C12-15 Alkyl Benzoate compounds, for example, can use natural palm-based C12-14 alkyl alcohol groups to which just enough synthetic C12-C15 alkyl alcohol groups are added to keep the INCI designation / name for the product being C12-15 Alkyl Benzoates, while still meeting all performance requirements of the fully synthetic C12-15 Alkyl Benzoates.

The mixtures of C12-15 Alkyl Benzoate compounds contains compounds that are specific palm-based C12 compounds, and contain a mixture of such naturally sourced compounds with some synthetic C12-15 Alkyl Benzoate compounds. For example, in certain embodiments it may be desirable to adjust the amount of certain Alkyl Benzoate compounds, which may be achieved by the addition of some amount of a single type of synthetic Alkyl Benzoate compound, e.g., C15, to an already existing composition of C12-15 Alkyl Benzoate compounds, thus, increasing the amount of C15 compounds in said composition. It is also possible to mix the naturally sourced Palm-Based C12-15 Alkyl Benzoate compounds with other known mixtures of C12-15 Alkyl Benzoate compounds, e.g., with some amount of FINSOLV^{®} TN.

The naturally sourced Palm-Based Alkyl Benzoate compounds contain specific C12 Alkyl Benzoate compounds. In such cases, the naturally sourced Palm-Based Alkyl Benzoate compounds may be readily used, or if industry standards or end use parameters mandate, some amount of C15 compounds may be added. For example, such a mixture may have the C12-14 Alkyl Benzoate compounds to C15 compounds ratio or 95/5 or the C12-14 Alkyl Benzoate compounds to C12-15 compounds ratio or 95/5. The amount of C15 compounds present or added may be however adjusted depending on desired use, e.g., to 12%, 10 %, 8%, 6%, 5.2%, 5%, 4%, 3%, 2% or 1%. In certain embodiments, preference is given to compound mixtures with less C15 compounds, e.g., 5-6%, e.g., less than 5.2%, or 3-5% or even less than 3%.

Preferably the amounts of the compounds are added during the manufacturing process, e.g., by selecting the amount of alkyl alcohols for use from, e.g., from palm-based sources and synthetic sources, e.g., for the C15 compounds. However, the desired mixtures may be achieved by separately preparing the C12-14 Alkyl Benzoate compounds from the C15 Alkyl Benzoate compounds and then later producing a mixture therefrom.

For example, in a specific preferred embodiment, the % of components in the final composition are as follows.

| | |
|---|---|
| C12-14 | 52.3% |
| C14-15 | 2.8% |
| Benzoic | 45.0% |

These values may be modified based on desired end use and/or desired characteristics.

The R groups, i.e., the degree of branching of the esters in the C12-15 Alkyl Benzoate compounds, affect various characteristics of the compounds, e.g., density, refractive index and especially pour point, meaning the more branching is present, the lower the pour point would be. So the proportions of C12, C13, C14 and 15 and the types and degrees of branching of each of these chain lengths may be important, and may well determine how much C15 compound one may desire to add to a composition containing primarily C12-C14 compounds.

The exact chemical composition of a mixture of C12-15 Alkyl Benzoate compounds may be determined by chromatographic analysis.

The naturally sourced palm-based Alkyl Benzoate compounds may be considered by those of ordinary skill in the art to be a "green" version of other known C12-15 Alkyl Benzoate compounds, e.g., Innospec's FINSOLV^{®} TN, and preferably, it meets the ISO 16128 Natural Standard. With such an advantage, the naturally sourced Alkyl Benzoate compounds would be of high interest for use in a large variety of products wishing to go more natural, e.g., low petro, instead of synthetic, typically petro based compounds.

Preferred properties of the novel Alkyl Benzoate compounds are as follows:

| PROPERTY | SPECIFICATION |
|---|---|
| APPEARANCE *@* 25°C | Clear Liquid |
| COLOR, APHA | 30 MAX |
| ACID VALUE, mg KOH/g | 0.10 MAX |
| MOISTURE, % wt | 0.10 MAX |
| SAP VALUE, mg KOH/g | 177-182 |
| ODOR | VERY MILD, PLEASANT, PRACTICALLY ODORLESS |
| REFRACTIVE INDEX @20°C | 1.4830-1.4870 |
| VISCOSITY, CPS @25°C | 50 Max. |
| SPECIFIC GRAVITY @ 25°C (g/mL) | 0.915-0.935 |
| IDENTIFICATION, IR | To Match Standard |
| RESIDUE ON IGNITION, % | 0.50 Max. |

Moreover noted is that the shelf life of the naturally sourced palm-based C12-15 Alkyl Benzoate compounds is high, e.g., four years, and preferably even more, e.g., 5 years.

The preparation of the C12-15 Alkyl Benzoate compounds is possible by known manufacturing methods that are readily apparent to those of ordinary skill in the art.

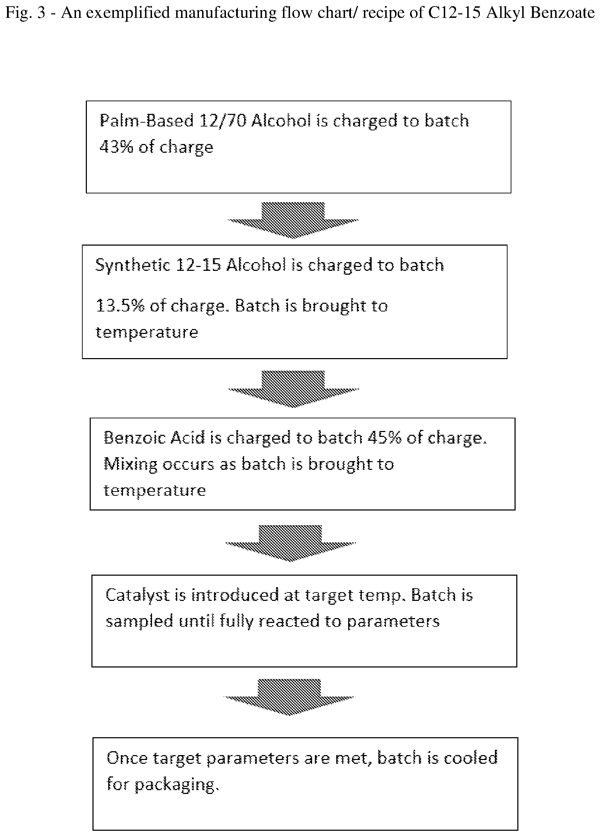

A manufacturing flow chart is provided in Fig. 3.

The flow chart provides a process where some natural as well as some synthetic alkyl alcohols are used for the production process.

Palm based alcohols, i.e., alkyl alcohols, are charged to a batch along with synthetic alkyl alcohols, which is brought to a temperature which is brought to a temperature to introduce esterification. Benzoic acid is charged to the batch and brought to a target temperature below the start of the esterification process. A suitable catalyst is introduced at the target temperature and the reaction is allowed to proceed at a suitable rate and the target reaction is allowed to proceed until target parameters are reached. The product is then cooled and can be packaged.

Preferably, the reaction proceeds to completion without reactants not having been left in the product, but some amount of trace reactants may remain, e.g., 0.5% free alcohol or less, e.g., 0.2% alcohol.

An option is to use natural C12-14 alcohol feedstock for the alcohol, e.g., palm kernel C12/C14 (codistilled, preferably), where the fatty alcohol would be about 46/16 (~74% C12 26% C14).

It is understood in the art that HPLC is not an exact art where each run would yield the exact same results. Thus, the HPLC peak area ratios given herein include values within a tolerance of, e.g., ± 10%, ±5% or ±2% and ±1%; and the HPLC peak values for retention time can also vary, e.g., by ± 0.2 minutes, ± 0.1 minutes, ± 0.05 minutes, or ± 0.02 minutes, or by a percentage difference allowance, e.g., ± 10% of the time, ± 5% of the time, ± 2% of the time, or ± 1% of the time.

### Brief Description of Drawings:

- Fig. 1: illustrates HPLC results for Sample A
- Fig. 2: illustrates HPLC results for Sample B
- Fig. 3: illustrates an exemplified manufacturing flow chart

### Examples:

Sample A was produced in accord with the recipe provided in Fig. 3.

Sample B is for illustrative purposes.

Results: Report of C12-15 Alkyl Benzoate Made with Palm-Based C12 versus Synthetic C15

Comparison study was done with two samples:
1. Natural C-12, sample A
2. FINSOLV^{®} TN from Innospec, sample B

FINSOLV^{®} TN has the following chemical description based on public information.

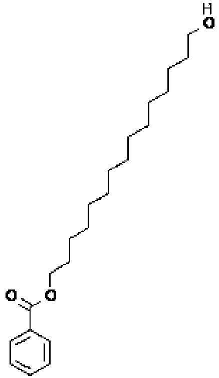

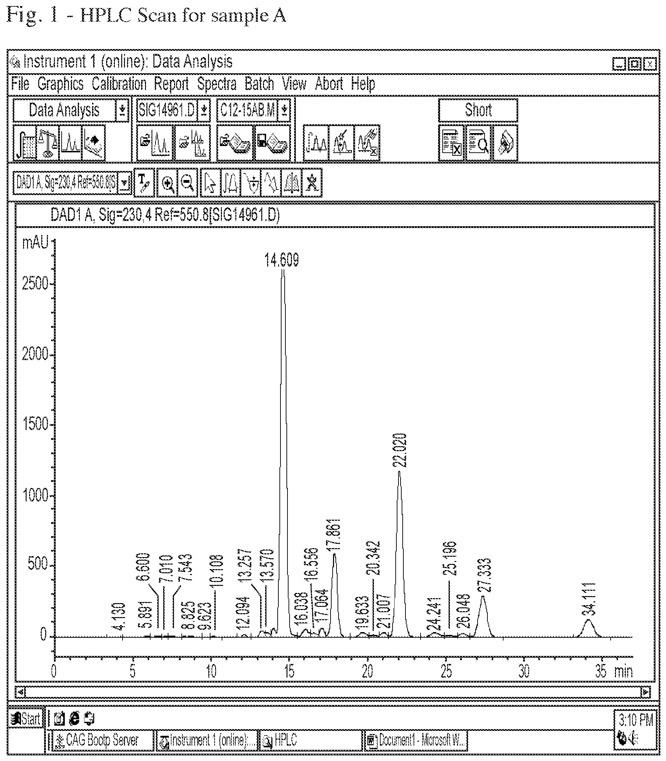

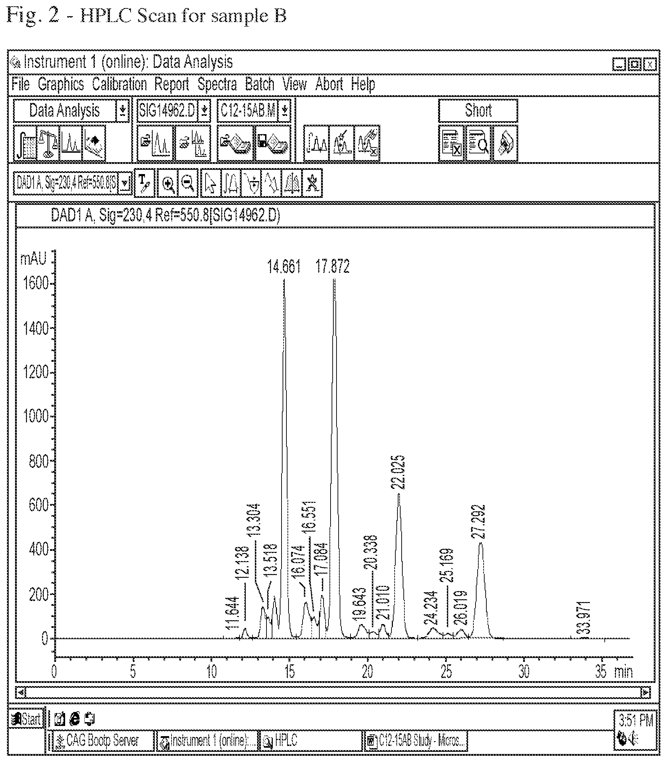

Composition of compounds by HPLC method @ 230nm provided the information illustrated in Figs. 1 and 2 for samples A and B, respectively.

**Table 1: The peak area ratios for five main peaks**

| peak# | Sample A | Sample B |
|---|---|---|
| 1 | 47.4 | 25.9 |
| 2 | 9.5 | 29.3 |
| 3 | 23.4 | 14.0 |
| 4 | 7.2 | 11.4 |
| 5 | 3.8 | 0.1 |

It is apparent from the scans and % compositions, the compound contributions from these two samples are very different due to different starting materials used.

**Table 2: Viscosity, dielectric constant, solubilities comparison table**

| | Viscosity (cst, 25°C) | Dielectric constant, RT | Solubilities (w/w), RT | | | |
|---|---|---|---|---|---|---|
| | | | %AVO | %BTZ | %DHHB | %EHT |
| Sample A | 12.97 | 3.94 | 12 | 8.2 | 8.2 | 1.8 |
| Sample B | 13.14 | 3.98 | 12 | 9.3 | 8.2 | 1.8 |

AVO = Avobenzone = Butyl Methoxydibenzoylmethane
BTZ = Bemotrizinol = Bis-Ethylhexyloxyphenol Methoxyphenyl Triazine
DHHB = Diethylamino Hydroxybenzoyl Hexyl Benzoate
EHT = Ethylhexyl Triazone

In the table above, very similar properties are contributed by these two samples. This means generally that their uses in industry can significantly overlap.

### Freeze point:

Sample A and sample B were kept in a freezer ( -15°C) overnight. Both were solid at room temperature right after freezer, and sample A turned to liquid faster than sample B. It means that sample A has higher freeze point then sample B.

**Table 3: Photostability - Experimentation was done in an Ethyl Acetate system**

| Formula# | JZ13-207 #1 | JZ13-207 #2 | JZ13-207 #3 | JZ13-207 #4 |
|---|---|---|---|---|
| Material tested | Sample A | Sample B | Sample A | Sample B |
| %Ethylhexyl methoxycinnamate | 7.5 | 7.5 | | |
| %Butyl Methoxydibenzoylmethane | | | 3.0 | 3.0 |
| %Material tested | 25.0 | 25.0 | 25.0 | 25.0 |
| PA-18, film former | 2.0 | 2.0 | 2.0 | 2.0 |
| Ethyl acetate | 65.5 | 65.5 | 70.0 | 70.0 |
| %remaining @306nm | 93.8 | 90.4 | | |
| %remaining @357nm | | | 69.5 | 71.7 |

In photostability test, sample A and sample B have very similar photostability for the two UV filters tested. Thus, one can just as effectively use the novel C12-15 Alkyl Benzoates Made with Palm-Based C12 as known products in the industry that are, e.g., fully synthetic.

**Table 4: Behavior in a typical product formulation**

| **Formulas of Comparing Performance** | | |
|---|---|---|
| **Formula#** | **Sample A** | **Sample B** |
| **Oil Phase Ingredients** | | |
| **C12-15 alkyl benzoates** | 30.00 | 30.00 |
| **Cetearyl alcohol** | 1.00 | 1.00 |
| **Glyceryl stearate** | 2.40 | 2.40 |
| **PEG-100 stearate** | 2.10 | 2.10 |

| **Water Phase Ingredients** | | |
|---|---|---|
| **Water** | 63.60 | 63.60 |
| **Xanthan Gum** | 0.30 | 0.30 |
| **Phenoxyethanol, Methylparaben, Ethylparaben, Butylparaben, Propylparaben, Isobutylparaben** | 0.60 | 0.60 |
| **Total** | 100.00 | 100.00 |
| **Viscosity (Brk, DV II, Sp.20, RVTD, after 24h at room temperature, 25C), CPS** | **5100** | **6900** |
| **pH** | **4.6** | **6.3** |

In the comparison work on real formulations,
- The batch containing 30% sample A had lower viscosity than 30%sample B
- The batch containing 30% sample A had lower pH than 30%sample B

pH may be adjusted by a variety of known ways if closer to neutral pH would be desired. Typical approaches for adjusting pH include cosmetic grade alkaline materials including metallic hydroxides and various amines.

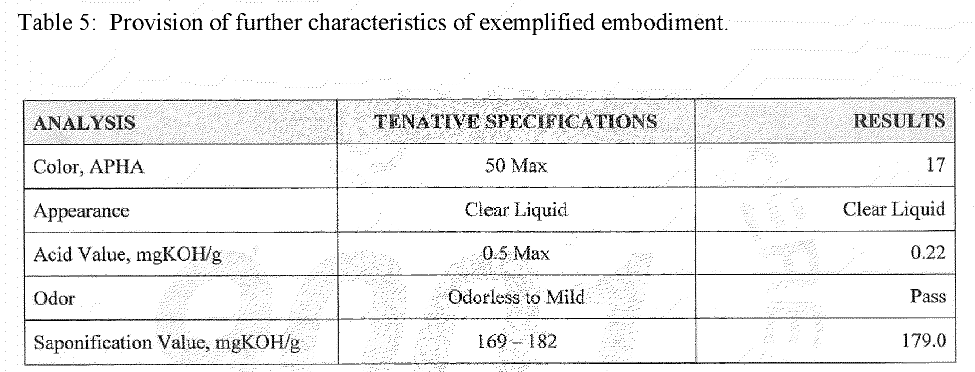

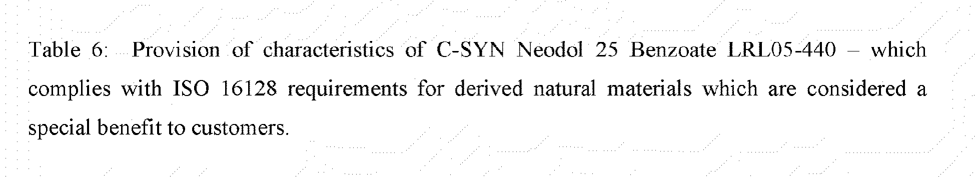

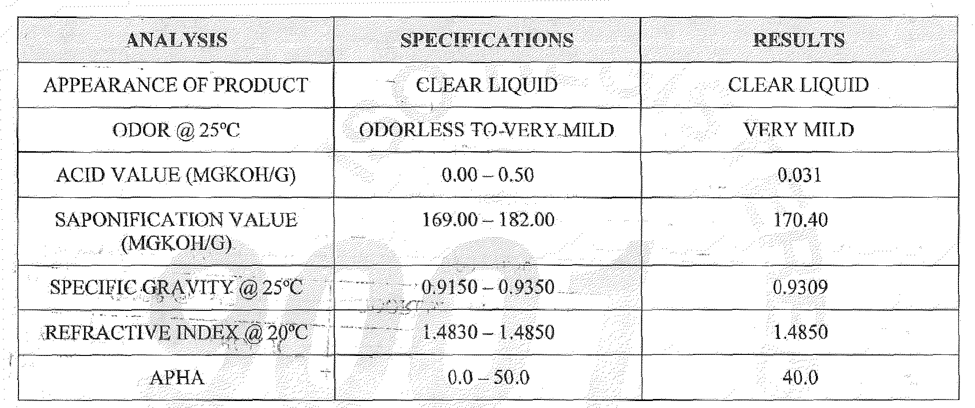

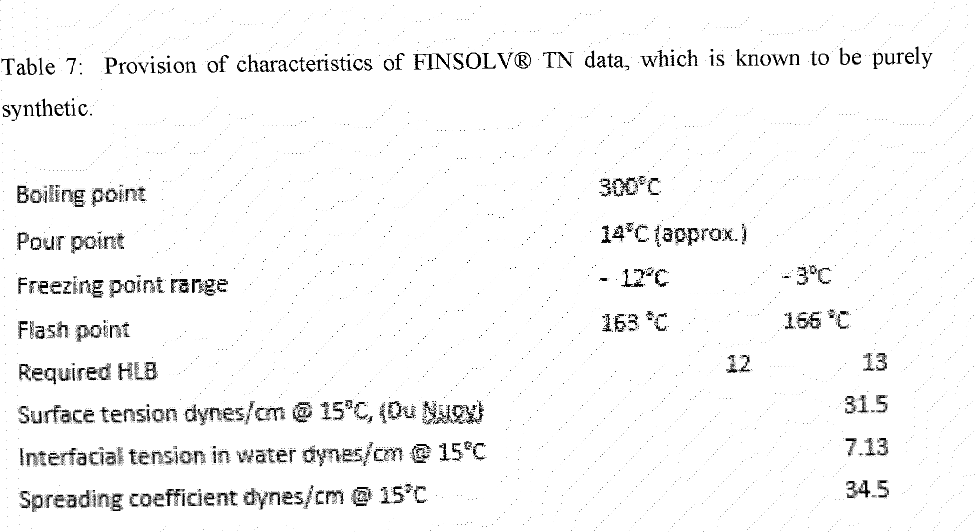

Thus, an objective is to achieve similar characteristics by the novel C12-15 Alkyl Benzoates to both the set of characteristics provided in Tables 6 and 7, so that customers would consider the novel C12-15 Alkyl Benzoates natural or natural enough, while providing the beneficial characteristics of fully synthetic C12-15 Alkyl Benzoates.

Further noted is that the solvency and low melting point of the novel C12-15 Alkyl Benzoates are such as needed to successfully replace FINSOLV^{®} TN as a one to one substitution in commercial products, thus, providing all the well-known benefits in many commercial products, while providing a more natural, greener, environmentally friendlier, lower synthetic, e.g., lower petro based sourced material, and thus a more costumer desired end product.

In sum, the novel C12-15 Alkyl Benzoates containing natural palm-based materials are surprisingly able to emulate the beneficial properties of synthetic C12-15 Alkyl Benzoates such as FINSOLV^{®} TN even when the amount of palm-based material is high, e.g., more than 60%, 70%, 71%, 75%, or even as high as 80% or above.

## Claims

1. An alkyl benzoate composition,
having peak area ratios for five main peaks by HPLC @ 230nm within a tolerance of ± 10% as follows
**[Table 1]**
| peak# | peak area ratio |
|---|---|
| 1 | 47.4 |
| 2 | 9.5 |
| 3 | 23.4 |
| 4 | 7.2 |
| 5 | 3.8 |
wherein the alkyl benzoate composition is produced by a process comprising:
charging a batch with 43% palm-based 12/70 alcohol and 13.5% synthetic C12-C15 alcohol and 45% benzoic acid,
bringing the batch to a predetermined temperature,
introducing a catalyst at the predetermined temperature,
and cooling the batch once free alcohol content is 0.5% or less.

2. A composition according to claim 1, to which C15 alkyl benzoate compounds are added.

3. A cosmetics, or beauty and/or body care product or fragrance, comprising the composition according to claim 1.

4. (New) A composition according to claim 1, wherein the tolerance is ±5%.

5. (New) A composition according to claim 1, wherein the tolerance is ±1%.

## Patentansprüche

1. Alkylbenzoat-Zusammensetzung
mit folgenden Peakflächenverhältnissen für fünf Hauptpeaks durch HPLC @ 230 nm innerhalb einer Toleranz von ± 10 %
**[Tabelle 1]**
| Peak-Nummer | Peakflächenverhältnis |
|---|---|
| 1 | 47,4 |
| 2 | 9,5 |
| 3 | 23,4 |
| 4 | 7,2 |
| 5 | 3,8 |
wobei die Alkylbenzoat-Zusammensetzung hergestellt wird durch ein Verfahren umfassend:
Laden einer Charge mit 43 % palmölbasiertem 12/70 Alkohol und 13,5 % synthetischem C12-C15 Alkohol und 45 % Benzoesäure,
Erhitzen der Charge auf eine vorbestimmte Temperatur,
Einbringen eines Katalysators bei Erreichen der vorbestimmten Temperatur,
und Abkühlen der Charge, sobald der freie Alkoholgehalt bei 0,5 % oder darunter liegt.

2. Zusammensetzung nach Anspruch 1, zu der C15 Alkylbenzoat-Verbindungen hinzugefügt werden.

3. Kosmetik oder Schönheits- und/oder Körperpflegeprodukt oder Duftstoff, umfassend die Zusammensetzung nach Anspruch 1.

4. (Neu) Zusammensetzung nach Anspruch 1, wobei die Toleranz ± 5 % beträgt.

5. (Neu) Zusammensetzung nach Anspruch 1, wobei die Toleranz ± 1 % beträgt.

## Revendications

1. Composition à base de benzoate d'alkyle,
dont les rapports de surface des cinq pics principaux, mesurés par HPLC à 230 nm, se situent dans une tolérance de ± 10 %, comme suit :
**[Tableau 1]**
| pic n° | rapport de surface de pic |
|---|---|
| 1 | 47,4 |
| 2 | 9,5 |
| 3 | 23,4 |
| 4 | 7,2 |
| 5 | 3,8 |
ladite composition à base de benzoate d'alkyle étant obtenue par un procédé comprenant les étapes suivantes :
charger un lot contenant 43 % d'alcool 12/70 à base d'huile de palme, 13,5 % d'alcool synthétique en C12-C15 et 45 % d'acide benzoïque,
porter le lot à une température prédéterminée,
introduire un catalyseur à la température prédéterminée
et refroidir le lot dès que la teneur en alcool libre est inférieure ou égale à 0,5 %.

2. Composition selon la revendication 1, à laquelle sont ajoutés des composés de benzoate d'alkyle en C15.

3. Produit cosmétique, produit de beauté et/ou de soins corporels ou parfum, comprenant la composition selon la revendication 1.

4. (Nouvelle revendication) Composition selon la revendication 1, où la tolérance est de ±5 %.

5. (Nouvelle revendication) Composition selon la revendication 1, où la tolérance est de ±1 %.
